# EUROPÄISCHE PATENTSCHRIFT

(11) **EP 2 414 017 B1**
(45) Veröffentlichungstag und Bekanntmachung des Hinweises auf die Patenterteilung: **06.08.2014**
(21) Anmeldenummer: 10721638.4
(22) Anmeldetag: 01.04.2010
(51) Int. Cl.: A61M 16/06

(54) **ATRAUMATISCHER NASENTUBUS FÜR DIE NICHTINVASIVE ATEMUNTERSTÜTZUNG**
ATRAUMATIC NASAL TUBE FOR NON-INVASIVE RESPIRATORY SUPPORT
MASQUE NASAL ATRAUMATIQUE POUR PRESSION POSITIVE CONTINUE NON INVASIVE

(30) Priorität: 04.04.2009 DE 102009016150
(43) Veröffentlichungstag der Anmeldung: 08.02.2012
(73) Patentinhaber: F. Stephan Gmbh Medizintechnik, 56412 Gackenbach (DE)
(72) Erfinder: MAINUSCH, Georg, 56076 Koblenz (DE); SCHALLER, Peter, 01326 Dresden (DE)
(74) Vertreter: Hannke, Christian
(86) Internationale Anmeldenummer: PCT/DE2010/000381
(87) Internationale Veröffentlichungsnummer: WO 2010/112017

(56) Entgegenhaltungen:
- WO-A1-2004/041341
- WO-A1-2008/007985
- DE-A1- 19 947 722
- US-A- 2 259 817
- US-A1- 2003 047 185
- US-A1- 2008 053 451

## Beschreibung

Die Erfindung betrifft einen atraumatischen Nasentubus für die nichtinvasive Atemunterstützung (NIV-CPAP).

Bei der maschinellen Beatmung oder Atemhilfe werden diejenigen Verfahren als nicht invasiv bezeichnet, bei denen das Atemgas nicht über einen in den Korper des Patienten eindringenden Endotrachealtubus, eine Trachealkanüle oder ein Tracheostoma, sondern über einen nasalen Tubus zugefuhrt wird.

Ein solcher nasaler Tubus oder Nasentubus verfügt über zwei kurze Nasenstutzen, die in die Nasenöffnungen eingeführt werden. Insbesondere bei fruhgeborenen Kindern kommt es wegen des äußerst sensiblen Nasenstegs und der Nasenschleimhaut nach längerer Liegezeit des Nasentubus zu Entzündungen, in schweren Fällen auch zu nekrotischen Veränderungen im Naseneingangsbereich.

Als Ursache hierfür ist unter anderem zu nennen, dass alle aus dem Stand der Technik vorbekannten nasalen Tuben ein gegenüber dem Nasensteg verlaufendes Querrohr aufweisen, aus dem unter einem Winkel von 90° in einem der Dicke des Nasenstutzens angepassten Abstand die Nasenstutzen austreten Im ungünstigsten Fall liegt dieses Querrohr am Nasensteg an und beeinträchtigt die Blutzirkulabon, was letztendlich zu Nasenstegnekrosen führt. Gleichermaßen kommt es im Naseninneren zu Verletzungen der Schleimhaut, wenn die Nasenstutzen entweder zu eng oder zu weit auseinander liegen. Aus diesem Grund werden von einigen Herstellern im neonatalen Bereich (Neugeborene) bis zu sechs verschiedene Größen angeboten, um eine optimale Anpassung an die geometrischen Verhältnisse der Nase zu gewährleisten.

Die US 2 259 817 A beschreibt einen atraumatischen Nasentubus für die nichtinvasive Atemunterstützung. Aus der US 2003/047185 A1 ist eine Atemunterstützungsvorrichtung bekannt. Diese weist einen Nasenkanal auf, um Atmungsgase, insbesondere einem Säugling, zuzuführen.

Die WO 2008/007985 beschreibt ebenfalls einen Atemunterstützungsapparat. Dabei wird insbesondere auch ein Geschirr zum Anlegen an einem Kopf dargestellt.

Die US 2008/053451 beschreibt eine Maskenvorrichtung zum Zuführen von Luft zu den Atmungswegen. Die WO 2004/041341 offenbart ebenfalls ein Kopfgeschirr für eine Beatmungsmaske.

Die Aufgabe der Erfindung besteht nunmehr darin, einen atraumatischen Nasentubus für die nichtinvasive Atemunterstützung vorzuschlagen, der einen hohen Tragekomfort ohne das Auftreten von Dermatitis oder Nekrosen sicherstellt, der problemlos an unterschiedlich ausgebildete Nasensepten und Kopfformen anpassbar ist und der kostengünstig zu fertigen ist.

Die Erfindung wird durch die Merkmale des unabhängigen Anspruchs 1 definiert.

Erfindungsgemäß umfasst der atraumatische Nasentubus für die nichtinvasive Atemunterstützung (NIV-CPAP) zumindest ein Zentralrohr, welches sich von dem Nasenrücken beabstandet vom Stirnbereich bis zur Nasenöffnung des Patienten erstreckt. Das erste Ende des Zentralrohrs ist dabei mit einem an der Stim des Patienten zu fixierenden Applikator zum Anschluss eines mit einer Beatmungsmaschine gekoppelten Schlauchsystems bestückt, und das zweite Ende des Zentralrohrs umfasst Nasenstutzen, die sichelförmig aus dem Zentralrohr austreten und in der Nasenöffnung münden.

Ein signifikanter Vorteil der Erfindung besteht darin, dass durch die sichelförmige Gestaltung der Nasenstutzen und das gegenüber dem Stand der Technik fehlende Querrohr sich erfindungsgemäß keine Komponente unmittelbar gegenüber dem Nasensteg befindet, wodurch die Entstehung von Nasenstegnekrosen konstruktiv ausgeschlossen ist. Weiterhin hat die Sichetform den Vorteil, dass die Nasenstutzen wegen ihrer großen Länge eine größere Elastizität besitzen, wodurch die Krafteinwirkungen auf die Nasenscheidewand reduziert werden mit dem Ergebnis einer verringerten Gefährdung der Nasenschleimhaut. Außerdem führt die Krümmung der Nasenstutzen zu einer größeren Flexibilität, wodurch größere Unterschiede in den Dicken der Nasenscheidewand überbrüdct werden und weniger verschiedene Tubengrößen benötigt werden.

Durch die modulare Bauweise des erfindungsgemäßen Nasentubus wird zum einen die Fertigung wesentlich vereinfacht. Zum anderen besteht die Möglichkeit, verschlissene Bauteile problemlos gegen neue Bauteile auszutauschen.

Die Fixierung des Applikators und damit mittelbar auch die des Zentralrohrs an der Stirn des Patienten erfolgt vorzugsweise unter Verwendung einer Stirnauflage, wobei der Applikator gegenüber der Stirnauflage längs in Richtung des Nasenrückens und/oder im Winkel bezogen auf die Stirnauflage verstellbar ist.

Die Verbindung zwischen der Stirnauflage und dem Applikator kann dabei als kraftschlüssige und/oder formschlüssige mechanische Verbindung ausgebildet sein oder durch eine Verbindung mittels Magnetkraft erfolgten.

Diese mechanische Verbindung gestattet eine Längsverstellung und /oder auch eine Winkelverstellung des Applikators zur Stirnauflage.

Zur Ausbildung einer Magnetverbindung im Falle lediglich einer Längsverstellung weisen die Stirnauflage und der Applikator jeweils einen magnetischen Abschnitt auf, die passfähig zueinander ausgebildet sind. Es hat sich als besonders praxistauglich erwiesen, wenn der Applikator hierzu einen im Querschnitt runden, flachen Dauermagneten aufweist und die Stirnauflage einen magnetischen Abschnitt in Form eines Ovals, Rechtecks oder einer Ellipse umfasst, welche(s) sich im Stirnbereich des Patienten entlang der Kopfhalbierenden des Patienten erstreckt, so dass eine stufenlose Höhenverstellung des Nasentubus bei unterschiedlichen Gesichtslängen besonders einfach realisiert werden kann. Durch die Magnetverbindung kann eine sehr einfache und schnelle Verbindung zwischen dem Nasentubus und der Stirnauflage erfolgen.

Die Mantelfläche des Zentralrohrs ist vorzugsweise zumindest partiell profiliert ausgebildet, wobei sich das Profil in Form einer Riffelung in Richtung der Längsachse des Zentralrohrs erstreckt, wodurch der Nasentubus zum Zwecke der Anpassung an die natürliche Krümmung der oberen Gesichtshälfte des Patienten besser elastisch verformt werden kann. Die Riffelung kann zum Zwecke der partiellen Verringerung der Wandstärke des Zentralrohrs dabei auf dessen Oberseite oder dessen Unterseite ausgebildet sein. Für ausgewählte Anwendungsfälle kann sich die Riffelung auch ringförmig um das Zentralrohr erstrecken.

Das vorzugsweise aus Kunststoff bestehende Zentralrohr weist im Querschnitt vorzugsweise ein nahezu rechteckiges Profil mit abgerundeten Kanten auf, wobei das Verhältnis von Breite zu Höhe etwa 2:1 beträgt.

Bei einer ersten bevorzugten Weiterbildung der Erfindung umfasst das Zentralrohr zusätzlich eingebettete Metalistrelfen, die zum Zwecke der Anpassung des Nasentubus an die natürliche Krümmung der oberen Gesichtshälfte des Patienten gemeinsam mit der in Richtung der Längsachse des Zentralrohrs sich erstreckenden Riffelung eine reversible Verformbarkeit des Zentralrohrs sicherstellen. Diese eingebetteten biegbaren Metallstreifen sind bevorzugt auf der Oberseite oder seiner Unterseite angeordnet und stoffschlüssig fest mit dem Zentralrohr verbunden, beispielsweise sind sie mit Kunststoff umspritzt. Anstelle der biegsamen Metallstreifen oder -bleche kann auch zumindest eine Drahtseele vorgesehen werden, die sich vorzugsweise in der Wandung der Stirnseiten des Zentralrohrs in dessen Längsrichtung erstrecken.

Bei einer weiteren bevorzugten Ausbildung der Erfindung verzichtet man auf die Fixierung der gewünschten Biegung des Zentralrohres mittels Metallstreifen oder Drahtseelen und sieht dafür noch eine zusätzliche Winkelverstellung im Applikator vor, mittels derer auch die Neigung des Zentralrohres gegenüber der Stirnauflage verändert werden kann. Diese Lösung hat den Vorteil, dass sowohl der Abstand als auch die Neigung des erfindungsgemäßen Nasentubus optimal an die geometrischen Verhältnisse zwischen Stirnauflage und Naseneingangsbereich angepasst werden können. In Folge des hochelastischen Zentralrohres werden dadurch alle Krafteinwirkungen seitens der Beatmungsschtäuche auf die Nasenlöcher weitestgehend reduziert.

Damit die Nasenstutzen gegenüber den Nasenlöchern noch besser fixiert werden können, wird eine formschlüssig auf das zweite Ende des Zentralrohres aufgesetzte Klemme vorgesehen. Diese enthält Fixationsmöglichkeiten für zusätzliche Bänder, die z. B. von der Stirnauflage kommend, das Zentralrohr auch in horizontaler Richtung fixieren.

Der Applikator umfasst mehrere Anschlussstutzen für die Zu- und Abführung von Medien, beispielsweise einen Anschlussstutzen für das zuströmende Inspirationsgas, einen Anschlussstutzen für das abströmende Expirationsgas, sowie einen mit einer Kappe verschlossenen Anschlussstutzen für eine Druckmesseinrichtung. Die vorgenannten Anschlussstutzen sind - je nach Erfordernis - jeweils vorzugsweise mit einem Schlauch gekoppelt, die ihrerseits mit einer Beatmungsmaschine verbunden sind.

Die Zuführung von Medikamenten mittels des erfindungsgemäßen Nasentubus erfolgt vorzugsweise unter Verwendung eines Anschlussstutzen bzw. Medikamentanschlusses, welcher durch das Aufstecken eines Adapters auf den Schlauch des Inspirationszweigs realisiert wird. Die Dosierung der entsprechenden Medikamente erfolgt bedarfsweise. Optional kann auch der Applikator einen derartigen Anschlussstutzen umfassen.

Zum Zwecke der Längenverstellung des Nasentubus sowie der Verbindung zwischen Zentralrohr und Applikator sind das erste Ende des Zentralrohres und der Anschlussstutzen für das Zentralrohr des Applikators als ineinander verschiebbare Fügepartner ausgebildet, wobei die Innenkontur des ersten Endes des Zentralrohrs und die Außenkontur des zugehörigen Anschlussstutzens des Applikators komplementär zueinander ausgebildet sind. Mit anderen Worten, der Applikator kann manuell auf den zugehörigen Anschlussstutzen des Zentralrohrs aufgesteckt werden. Mittels der weiteren Anschlussstutzen des Applikators wird die Verbindung zu dem inspiratorischen und expiratorischen Beatmungsschlauch hergestellt.

Innerhalb des Zentralrohrs eines atraumatischen Nasentubus ist eine in Richtung seiner Längsachse sich erstreckende median angeordnete Trennwand platziert, die das Zentralrohr in eine erste Strömungskammer für das Inspirationsgas und in eine zweite Strömungskammer für das Expirationsgas unterteilt. Diese, eine vorzeitige Vermischung von Inspirationsgas und Expirationsgas verhindernde Trennwand erstreckt sich ausgehend vom ersten Ende des Zentralrohrs, also unmittelbar zwischen dem Anschlussstutzen für das zuströmende Inspirationsgas und dem Anschlussstutzen für das abströmende Expirationsgas, bis etwa 5 bis 15 mm vor dem zweite Ende des Zentralrohres und endet dort. Ein Nachteil der Trennwand ist jedoch die damit verbundene erhöhte Steifigkeit des Nasentubus gegenüber einer erwünschten Biegung. Bei einer alternativen Ausgestaltung ist zur Vermeidung dieses Nachteils die Trennwand teilweise unterbrochen ausgebildet oder wird aus mehreren stirnseitig voneinander beabstandeten Stegen gebildet. Wie in aufwändigen Messreihen aufgezeigt wurde, ist bei den geometrischen Verhältnissen des Zentralrohres und den gewählten Volumenströmen nicht mit einer CO₂-Rückatmung zu rechnen, so dass in der besonders einfachen und erfindungsgemäßen Ausführung der Erfindung auf die Trennwand ganz verzichtet werden kann.

Die Nasenstutzen weisen vorzugsweise ringförmige Verringerungen der Wandstärke auf. Diese ringförmige Verringerung der Wandstärke tragen zur Erzielung einer größeren Elastizität der Nasenstutzen bei.

Die Nasenstutzen können ein rundes oder bevorzugt ein der Form des Nasenloches angepasstes dreieckiges Profil aufweisen. Bei der Verwendung eines im Querschnitt dreieckigen Profils wird eine größere Austrittsfläche, verbunden mit einem geringeren Strömungswiderstand und einer besseren Abdichtung gegenüber der Nasenscheidewand, erzielt.

Bei einer vorteilhaften Weiterbildung der Erfindung verringert sich der innere Querschnitt der Nasenstutzen ausgehend vom Zentralrohr bis zur in der Nasenöffnung platzierten Mündung. Es wird somit verhindert, dass durch die größere Länge der Nasenstutzen der Strömungswiderstand zunimmt.

Des Weiteren besteht die Möglichkeit, im Bereich des zweiten Endes des Zentralrohrs einen Klipphalter als Winkelverstellung vorzusehen, mittels derer auch die Neigung des Zentralrohres gegenüber der Stirnauflage verändert werden kann.

Die signifikanten Vorteile und Merkmale der Erfindung gegenüber dem Stand der Technik sind im Wesentlichen:
■ der nur aus wenigen Bauteilen modular aufgebaute Nasentubus ist kostengünstig herstellbar und leicht zu montieren,
■ durch die sichelförmige Gestaltung der Nasenstutzen und das gegenüber dem Stand der Technik fehlende Querrohr des Nasentubus befindet sich keine Komponente unmittelbar gegenüber dem Nasensteg, wodurch die Entstehung von Nasenstegnekrosen konstruktiv ausgeschlossen ist,
■ durch den im Stirnbereich des Patienten entlang der Kopfhalbierenden des Patienten sich erstreckenden magnetischen Abschnitt der Stirnauflage kann eine stufenlose Höhenverstellung des Nasentubus zur Anpassung an unterschiedliche Gesichtslängen besonders einfach realisiert werden,
■ durch die optionale Möglichkeit einer Winkelverstellung kann der Nasentubus auch bezüglich des Winkels an die Gesichtsgeometrie weitestgehend angepasst werden,
■ der Nasentubus ist unter Verwendung seines mehrere Anschlussstutzen aufweisenden Applikators mit verschiedenen Beatmungssystemen einsetzbar,
■ mittels der sich in Richtung der Langsachse des Zentralrohrs erstreckenden Riffelung und/oder der eingearbeiteten Drahtseelen kann der Nasentubus zum Zwecke der Anpassung an die natürliche Krümmung der oberen Gesichtshälfte des Patienten elastisch verformt werden,
■ der Applikator weist optional zusätzlich einen Anschlussstutzen bzw. eine Schnittstelle für Medikamente auf,
■ durch die Verwendung von im Querschnitt dreieckigen Proflen der Nasenstutzen wird eine größere Austrittsfläche, verbunden mit einem geningeren Strömungswiderstand und einer besseren Abdichtung gegenüber der Nasenscheidewand erzielt und
■ der Nasentubus weist optional einen im Bereich des Zentralrohrs oder Applikators platzierten Klipphalter auf, um seinen Einsatz auch bei agilen Babys zu ermöglichen.

Die Ziele und Vorteile dieser Erfindung sind nach sorgfältigem Studium der nachfolgenden ausführlichen Beschreibung der hier bevorzugten, nicht einschränkenden Beispielausgestaltungen der Erfindung mit den zugehörigen Zeichnungen besser zu verstehen und zu bewerten. Von denen zeigen:
- Fig. 1:: eine perspektivische Darstellung des Nasentubus mit Klipphalter zur Winkelverstellung als Komplettsystem,
- Fig. 2:: eine perspektivische Darstellung des Nasentubus mit Klipphalter zur Winkelverstellung und mit zusätzlicher Stirnauflage unmittelbar vor der Montage,
- Fig. 3:: eine perspektivische Darstellung des Nasentubus mit Klipphalter zur Winkelverstellung im Zusammenwirken mit der Stirnaufiage im Beatmungszustand.
- Fig. 4:: eine perspektivische Darstellung des Nasentubus mit Magnetverstellung als Komplettsystem,
- Fig. 5:: eine Explosionsdarstellung des Nasentubus und Maske
- Figuren 6.1 bis 6.3: eine Stirnauflage in verschiedenen Ansichten.

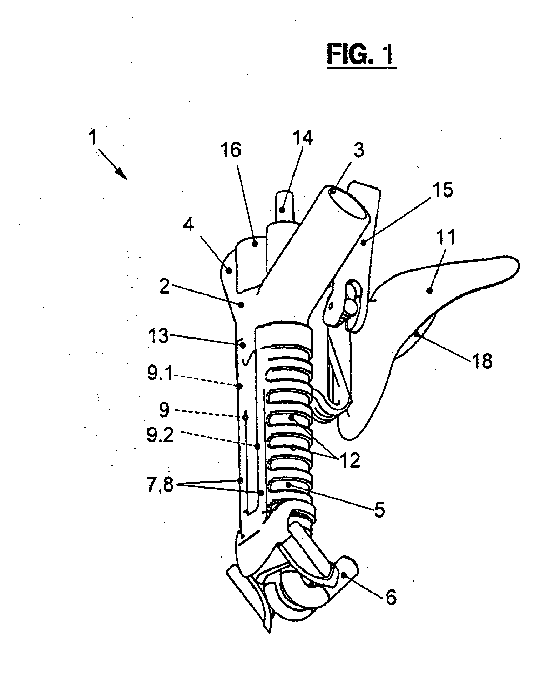

Die Figur 1 illustriert einen erfindungsgemäßen atraumatischen Nasentubus 1 mit zusätzlicher Winkelverstellung als Perspektivdarstellung für die nichtinvasive Atemunterstützung (NIV-CPAP). Der Nasentubus 1 umfasst im dargestellten Beispiel ein Zentralrohr 5, welches sich von dem Nasenrücken beabstandet vom Stirnbereich bis zur Nasenöffnung des Patienten erstreckt, wobei das erste Ende des Zentralrohrs mit einem an der Stirn des Patienten zu fixierenden Applikator zum Anschluss eines mit einer Beatmungsmaschine gekoppelten Schlauchsystems bestückt ist, und das zweite Ende des Zentralrohrs Nasenstutzen 6 umfasst, die sichelförmig aus dem Zentralrohr 5 austreten und in der Nasenöffnung münden. Der Applikator 2 weist einen Anschlussstutzen 3 für das zuströmende Inspirationsgas, einen Anschlussstutzen 4 für das anströmende Expirationsgas, einen Anschlussstutzen 14 für das Zentralrohr 5 und zwei Anschlussstutzen 13 für zuzuführende Medikamente auf. Zum Zwecke der Längenverstellung des Nasentubus 1 und zum Zwecke der Kopplung sind das erste Ende des Zentralrohres 5 und der Anschlussstutzen 14 für das Zentralrohr 5 des Applikators 2 als ineinander verschiebbare Fügepartner ausgebildet, wobei die Innenkontur des ersten Endes des Zentralrohrs 5 und die Außenkontur des Anschlussstutzens 14 des Applikators 2 komplementär zueinander ausgebildet sind. Die Mantelfläche des Zentralrohrs 5 ist zumindest partiell profiliert ausgebildet, wobei sich das Profil in Form einer Riffelung 12 in Richtung der Längsachse des Zentralrohrs 5 erstreckt. Diese Riffelung 12 umfasst quer zur Längsachse des Zentralrohrs 5 verlaufende Materialverdickungen bzw. Materialausnehmungen. Dadurch wird trotz der Tatsache, dass das Zentralrohr 5 aus einem elastischen Kunststoff gefertigt ist, eine noch höhere Elastizität ersielt, so dass der Nasentubus 1 optimal an die natürliche Krümmung der oberen Gesichtshälfte des Patienten angepasst werden kann. Innerhalb des Zentralrohrs 5 ist eine sich in Richtung seiner Längsachse erstreckende median angeordnete Trennwand 9 platziert, die das Zentralrohr 5 in eine erste Strömungskammer 9.1 für das Inspirationsgas und in eine zweite Strömungskammer 9.2 für das Expirationsgas unterteilt. Diese Trennwand 9 endet etwa 5 bis 15 mm vor dem zweiten Ende des Zentralrohres 5. Die aus dem Zentralrohr 5 sichelförmig austretenden beiden Nasenstutzen 6 weisen jeweils ein der Form des Nasenlochs angepasstes dreieckiges Profil auf. Der innere Querschnitt der Nasenstutzen 6 verringert sich dabei ausgehend vom Zentralrohr 5 bis zur in der Nasenöffnung platzierten Mündung. Beide Nasenstutzen 6 verlaufen im Wesentlichen parallel zueinander und erstrecken sich - bei angelegtem Nasentubus am Patienten - ausgehend vom zweiten Ende des Zentralrohrs 5 über die Nasenspitze bis zu hin zu den Nasenöffnungen. Das Zentralrohr 5 ist im Querschnitt im Wesentlichen rechteckig mit abgerundeten Kanten ausgebildet, wobei das Verhältnis von Breite zu Höhe etwa 2:1 beträgt. Im Bereich der schmalen Stirnseiten des Zentralrohrs 5 sind beidseitig jeweils eine Drahtseele eingebettet bzw. von Kunststoff umspritzt, um dem Zentralrohr 5 zur Anpassung an die Krümmung der verschiedenartig ausgebildeten oberen Gesichtshälften der Patienten ein reversibles Verformungsmögert zu geben. Zum Zwecke der Winkelverstellung ist im Bereich des Applikators 2 ein Klipphalter 15 platziert, mit dem einerseits die Längenverstellung des Nasentubus 1 an die Gesichtslänge und andererseits die Neigung des Näsentubus 1 zum Zwecke der Anpassung an die Krümmung des Gesichts des Patienten realisiert werden kann.

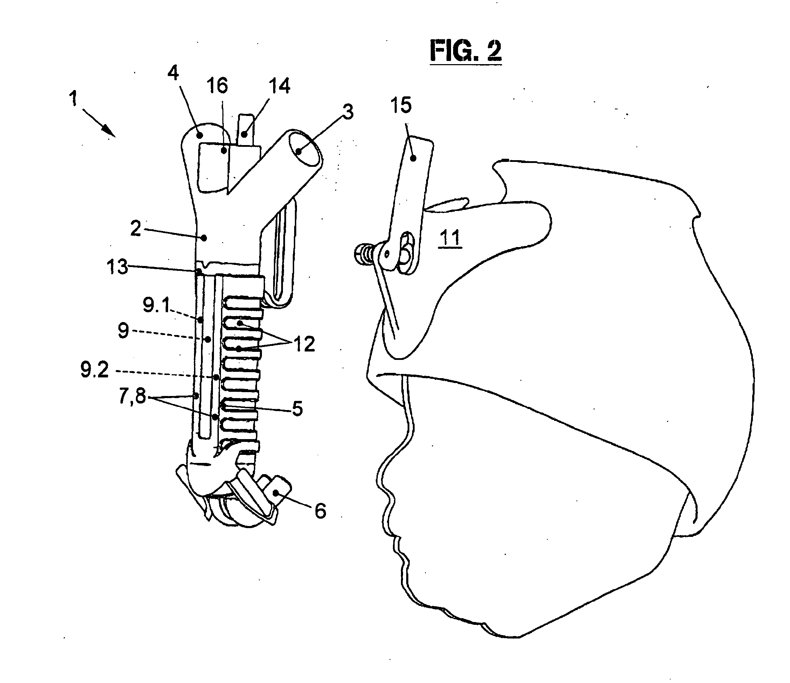

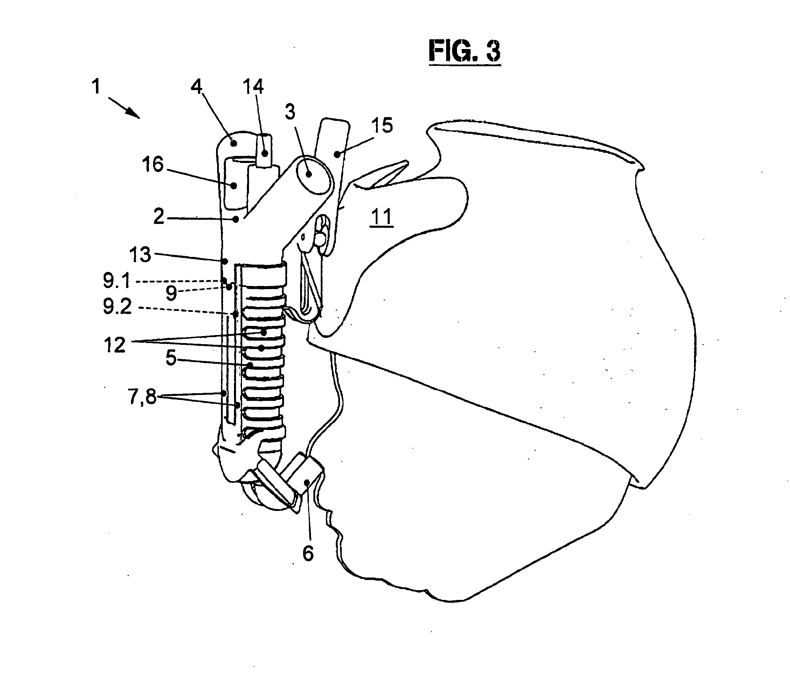

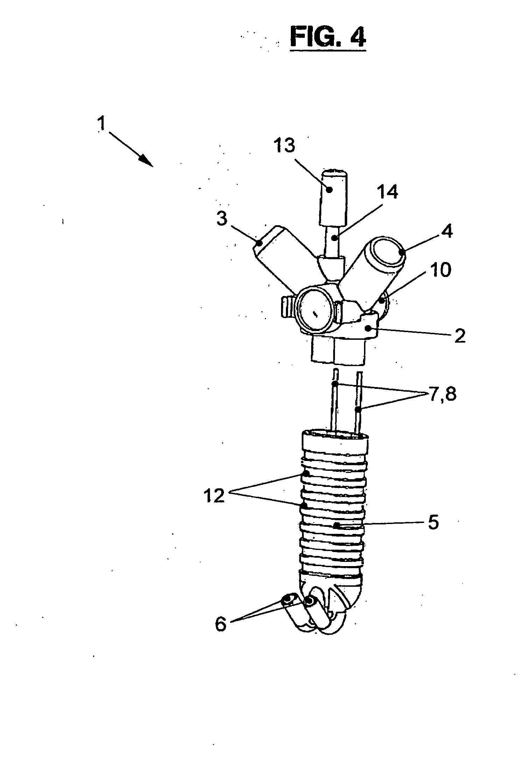

Die Fig. 2 zeigt eine perspektivische Darstellung des Nasentubus und der Stirnauflage unmittelbar vor der Montage, wobei die Stirnauflage auf einer angedeuteten medizinischen Kappe oder Mütze des Patienten platziert ist und mittels derer die Stirnauflage 11 mittelbar am Kopf des Patienten arretiert wird. Anstelle der Kappe oder Mütze kann optional auch ein Band mit einem Klettverschluss eingesetzt werden, welches um den Kopf des Patienten verspannt wird. Die Stirnauflage 11 dient zur Fixierung des Applikators 2 und damit des Nasentubus 1 an der Stirn des Patienten unter Verwendung des besagten Bandes oder eines Klettverschlusses 18. Der Klettverschlusses 18 bildet einen ersten Fixpunkt des Nasentubus 1 aus. Der zweite Fixpunkt wird durch die sichelförmig aus dem Zentralrohr 5 austretenden Nasenstutzen 6 gemäß der Figuren 3 und 4 erzielt, die in der Nasenöffnung münden.

In der Fig. 3 ist eine perspektivische Darstellung des Nasentubus 1 im Zusammenwirken mit der Stirnauflage 11 im Beatmungszustand gezeigt. Wie ersichtlich, erstreckt sich der erfndungsgemäße Nasentubus 1 ausgehend von der Stirn bis hin zur Nasenöffnung des Patienten. Der Nasentubus 1 weist besonders vorteilhaft nur zwei Fixpunkte am Patienten auf, nämlich einen ersten Fixpunkt im Bereich des am ersten Ende des Zentralrohrs 5 angeordneten Applikators 2 und einen zweiten Fixpunkt, der am zweiten Ende des Zentralrohrs im Form der beiden Nasenstutzen 6 ausgebildet ist. Bis auf die in der Nasenöffnung mündenden Nasenstutzen 6 kontaktiert keiner der Komponenten des erfindungsgemäßen Nasentubus, 1 unmittelbar die ungeschützte Haut im Naseneingangsbereich des Patienten. Dadurch werden Druckstellen und Nekrosen wirkungsvoll verhindert. Der dennoch sehr nah am Kopf des Patienten angeordnete Nasentubus 1 kann mit wenigen Handgriffen in die ewünschte Position gebracht und an die unterschiedlichen Gesichtsformen durch eine Längenverstellung und Einstellung der Krümmung des Zentralrohrs 5 problemlos angepasst werden.

Bei allen zuvor beschriebenen Figuren 1 bis 3 umfasst der Nasentubus 1 einen Küpphalter 15 als Winkelverstellung, mittels derer auch die Neigung des Zentralrohres 5 gegenüber der Stirnauflage 11 verändert werden kann. Diese Lösung hat den Vorteil, dass sowohl der Abstand als auch die Neigung des erfindungsgemäßen Nasentubus 1 optimal an die geometrischen Verhältnisse zwischen der Stirnauflage 11 und Naseneingangsbereich angepasst werden können. In Folge des hochelastischen Zentralrohres 5 werden dadurch alle Krafteinwirkungen seitens der nichtdargestellten Beatmungsschläuche auf die Nasenlöcher weitestgehend reduziert. Damit die Nasenstutzen 6 gegenüber den Nasenlöchern noch besser fixiert werden können, wird eine formschlüssig auf das zweite Ende des Zentralrohres 5 aufgesetzte Klemme vorgesehen. Diese enthält Fixationsmöglichkeiten für zusätzliche Bänder, die z. B. von der Stirnauflage kommend, das Zentralrohr 5 auch in horizontaler Richtung fixieren.

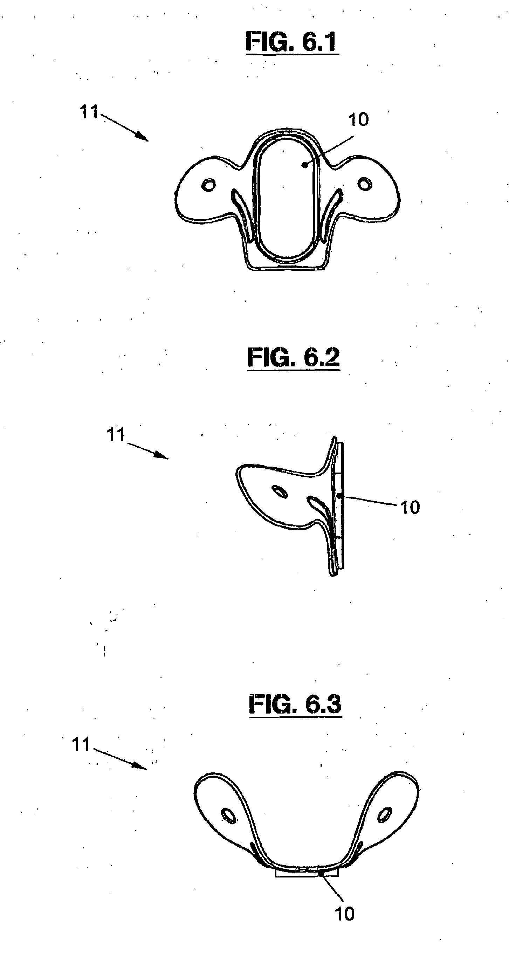

Die Fig. 4 zeigt eine perspektivische Darstellung des Nasentubus 1 mit Magnetverstellung als Komplettsystem. Der Nasentubus 1 entspricht im Wesentlichen den in den Figuren 1 bis 3 beschriebenen Nasentubus 1 mit dem Unterschied, dass hier die im Zentralrohr 5 platzierten Metallstreifen 7 bzw. Drahtseelen 8 sichtbar sind. Die paarweise ausgebildeten Metallstreifen 7 bzw. Drahtseelen 8 erstrecken sich hierbei in Richtung der Längsachse des Zentralrohrs 5. Ein weiterer wesentlicher Unterschied gegenüber der zuvor beschriebene Ausgestaltung besteht darin, dass die Stirnauflage 11 im Ausführungsbeispiel gemäß Fig. 4 keinen Klipphalter 15 zur Winkelverstellung umfasst. Vielmehr kommt eine Magnetverbindung zum Einsatz, mittels derer der Applikator 2 an der Stirnauflage 11 fixiert wird. Die Magnetverbindung wird gebildet durch einen im Querschnitt kreisförmigen magnetischen Abschnitt 10, der passfähig zu dem magnetischen Abschnitt der Stirnauflage 11 gemäß der Figuren 6.1 bis 6.3 ist. Der magnetische Abschnitt 10 der Stirnauflage 11 und der magnetische Abschnitt 10 des Applikators 2 bilden als Magnetverbindung einen ersten Fixpunkt des Nasentubus 1 aus. Bei einer nichtdargestellten Weiterbildung der Erfindung ist der magnetische Anschnitt 10 der Stirnauflage 11:als Oval, Ellipse oder Rechteck ausgebildet, welche(s) sich im Stirnbereich entlang der Kopfhalbierenden des Patienten erstreckt, so dass eine stufenlose Höhenverstellung des Nasentubus 1 bei unterschiedlichen Gesichtslängen realisiert werden kann.

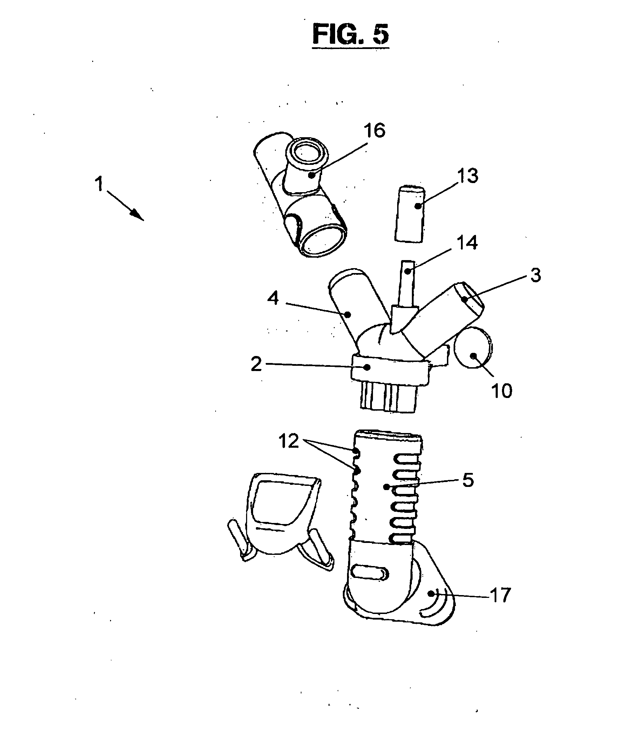

Die Fig. 5 zeigt eine Explosionsdarstellung des Nasentubus 1. Als wesentliche Merkmale des hier gezeigten Nasentubus 1 sind die am zweiten Ende des Zentralrohrs 5 platzierte elastischen Maske 17, die anstelle der sichelförmigen Nasenstutzen 6 eingesetzt wird, sowie der Anschlussstutzen 16 für die Medikamentenzufuhr zu nennen, welcher nicht unmittelbar am Applikator 1, sondern an einem Adapterstück im Inspirationszweig bzw. Inspirationsschlauch angeordnet ist. Die Maske 17 ist - ebenso wie die Nasenstutzen 6 - unmittelbar mit dem Zentralrohr 5 verbunden, so dass aus montagetechnischer Sicht beim Einsatz dieser Maske 17, nur das Zentralrohr 5 ausgetauscht werden muss, an welchem die gewünschten Bauteile, nämlich die Maske 17 oder die Nasenstutzen 6, wahlweise platziert sind. Diese beiden unterschiedlichen Ausgestaltungen sind sehr deutlich dem Vergleich zwischen der Fig. 4 und Fig.5 zu entnehmen.

Die Figuren 6.1 bis 6.3 illustrieren eine Stirnauflage 11 in verschiedenen Ansichten. Die auf einer Kopfmütze oder Kopfkappe platzierte Stirnauflage 11 ist im Wesentlichen schmetterlingsförmig ausgebildet, wobei die beiden Flügel entsprechend der Kopfform des Patienten gekrümmt sind. Die Krümmung wird erzielt durch die Ausbildung der Stirnauflage 11 aus Kunststoff. Zwischen den beiden Flügeln ist ein im Querschnitt ovaler magnetischer Abschnitt 10 platziert, der mit dem magnetischen Abschnitt 19 des Applikators 2 gemäß Fig. 5 korrespondiert; wodurch der Applikator 2 an der Stirnauflage 11 magnetisch gehaltert ist. Durch die ovale Ausführung des magnetischen Abschnitts 10 kann der Nasentubus 1 besonderes einfach an die Länge des Gesichts des Patienten angepasst werden. Die Flügel umfassen ferner (jeweils) einen Klettverschluss, der an einer auf dem Kopf des Patienten zu platzierenden Mütze bzw. an einem Kopfband arretiert wird.

### LISTE DER BEZUGSZEICHEN

- 1: Nasentubus
- 2: Applikator
- 3: Anschlussstutzen für Inspirationsschlauch
- 4: Anschlussstutzen für Expirationsschlauch
- 5: Zentralrohr
- 6: Nasenstutzen
- 7: Metallstreifen
- 8: Drahtseele
- 9: Trennwand
- 9.1: erste Strömungskammer
- 9.2: zweite Strömungskammer
- 10: magnetischer Abschnitt
- 11: Stirnauflage
- 12: Riffelung
- 13: Kappe für Druckmessstutzen
- 14: Anschlussstutzen für Druckmesseinrichtung
- 15: Klipphalter
- 16: Anschlussstutzen für Medikamentenzufuhr
- 17: Maske
- 18: Klettverschluss

## Patentansprüche

1. Atraumatischer Nasentubus (1) für die nichtinvasive Atemunterstützung (NIV-CPAP), zumindest umfassend ein vom Inspirations- und Expirationsgas durchströmtes Zentralrohr (5), welches sich von dem Nasenrücken beabstandet vom Stirnbereich bis zur Nasenöffnung des Patienten erstreckt, wobei das erste Ende des Zentralrohres (5) in einem an der Stirn des Patienten zu fixierenden Applikator (2) zum Anschluss eines mit einer Beatmungsmaschine gekoppelten Schlauchsystems mündet, und das zweite Ende des Zentralrohrs (5) Nasenstutzen (6) umfasst,
**dadurch gekennzeichnet, dass**
die Nasenstutzen (6) sichelförmig nach oben aus dem Zentralrohr (5) austreten und in die Nasenöffnung münden, so dass sich keine Komponente unmittelbar gegenüber dem Nasensteg befindet und dass das Zentralrohr (5) unter vollständigem Verzicht auf eine, eine vorzeitige Vermischung von zuströmendem Inspirationsgas und abströmendem Expirationsgas verhindernde, Trennwand ausgebildet ist.

2. Atraumatischer Nasentubus (1) nach Anspruch 1,
**dadurch gekennzeichnet, dass**
die Fixierung des Applikators (2) an der Stirn des Patienten unter Verwendung einer Stirnauflage (11) erfolgt, wobei der Applikator (2) gegenüber der Stirnauflage längs in Richtung des Nasenrückens und/oder im Winkel bezogen auf die Stirnauflage (11) verstellbar ist.

3. Atraumatischer Nasentubus (1) nach einem der Ansprüche 1 und 2,
**dadurch gekennzeichnet, dass**
die Fixierung des Applikators (2) an der Stirnauflage (11) auf magnetische Weise erfolgt, wobei die Stirnauflage (11) und der Applikator (2) jeweils einen magnetischen Abschnitt (10) umfassen, die passfähig zueinander ausgebildet sind und eine stufenlose Höhenverstellung des Nasentubus (1) ermöglichen.

4. Atraumatischer Nasentubus (1) nach Anspruch 3,
**dadurch gekennzeichnet, dass**
der magnetische Abschnitt (10) der Stirnauflage (11) als Oval, Ellipse oder Rechteck ausgebildet ist, welche(s) sich im Stirnbereich des Patienten entlang der Kopfhalbierenden des Patienten erstreckt, so dass eine stufenlose Höhenverstellung des Nasentubus (1) bei unterschiedliche Gesichtslängen realisiert werden kann.

## Claims

1. An atraumatic nasal tube (1) for non-invasive respiratory support (NIV CPAP), at least comprising a central tube (5) through which the inspiration and expiration gas flows and which extends at a distance from the bridge of the nose from the forehead region as far as the nasal opening of the patient, wherein the first end of the central tube (5) opens into an applicator (2) to be fixed to the forehead of the patient for the attachment of a hose system coupled to a respiratory machine, and the second end of the central tube (5) comprises nasal connecting pieces (6), **characterized in that** the nasal connecting pieces (6) emerge from the second end of the central tube (5) and extend upwards in a sickle shape into the nasal opening, so that no component is present immediately opposite the bridge of the nose, and the central tube (5) is designed so as to abandon completely a partition wall which prevents a premature mixing of inflowing inspiration gas and outflowing expiration gas.

2. An atraumatic nasal tube (1) according to claim 1, **characterized in that** the fixing of the applicator (2) to the forehead of the patient is carried out using a forehead pad (11), wherein the applicator (2) is capable of being adjusted with respect to the forehead pad lengthwise in the direction of the bridge of the nose and/or at an angle to the forehead pad (11).

3. An atraumatic nasal tube (1) according to one of claims 1 and 2, **characterized in that** the fixing of the applicator (2) to the forehead pad (11) is carried out in a magnetic manner, wherein the forehead pad (11) and the applicator (2) comprise one magnetic portion (10) in each case, these magnetic portions (10) being designed capable of fitting to each other and permitting a continuous vertical adjustment of the nasal tube (1).

4. An atraumatic nasal tube (1) according to claim 3, **characterized in that** the magnetic portion (10) of the forehead pad (11) is designed in the form of an oval, ellipse or rectangle which extends in the forehead area of the patient along the bisectrix of the head of the patient, so that a continuous vertical adjustment of the nasal tube (1) can be carried out in the case of different lengths of the face.

## Revendications

1. Masque nasal atraumatique (1) pour pression positive continue non invasive (VNI-CPAP), comprenant au moins un conduit central (5) à travers lui passent des gaz d'inspiration et d'expiration et qui s'étend espacé de l'arête du nez, de la région du front jusqu'à la narine du patient, la première extrémité du conduit central (5) débouchant dans un applicateur (2) à fixer contre le front du patient pour le branchement d'un système de tuyau connecté à un appareil respiratoire, et la deuxième extrémité du conduit central (5) comprenant des tubulures nasales (6),
**caractérisé en ce que**
les tubulures nasales (6) sortent de la deuxième extrémité du conduit central (5) et remontent chacune en forme de faucille vers la narine, si bien qu'aucun composant ne se trouve directement en face de la racine du nez et que le conduit central (5) est réalisé en renonçant complètement à une cloison de décrochage empêchant un mélange précoce du gaz d'inspiration affluent et du gaz d'expiration effluent.

2. Masque nasal atraumatique (1) selon la revendication 1, **caractérisé en ce que**
la fixation de l'applicateur (2) contre le front du patient resulte en recourant à un appui de front (11), l'applicateur (2) étant réglable longitudinalement vers l'arête du nez en face de l'appui de front et/ou angulairement par rapport à l'appui de front (11).

3. Masque nasal atraumatique (1) selon l'une quelconque des revendications 1 et 2, **caractérisé en ce que**
la fixation de l'applicateur (2) contre l'appui de front (11) est obtenue magnétiquement, l'appui de front (11) et l'applicateur (2) comprenant chacun une pièce magnétique (10) réalisée de manière à pouvoir être ajustée par rapport à la pièce correspondante et permettant un réglage continu en hauteur du masque nasal (1).

4. Masque nasal atraumatique (1) selon la revendication 3, **caractérisé en ce que**
la pièce magnétique (10) de l'appui de front (11) est réalisée dans une forme ovale, elliptique ou rectangulaire, qui s'étend dans la région du front du patient le long de l'arête du front du patient, de manière à permettre un réglage continu en hauteur du masque nasal (1) pour différentes longueurs de visage.
